# EUROPÄISCHE PATENTANMELDUNG

(11) **EP 1 839 566 A1**
(43) Veröffentlichungstag der Anmeldung: **03.10.2007**
(21) Anmeldenummer: 06006502.6
(22) Anmeldetag: 29.03.2006
(51) Int. Cl.: A61B 5/00

(54) **Verfahren und Anordnung zur Überwachung eines medizinischen Gerätes**

(71) Anmelder: F. HOFFMANN-LA ROCHE AG, 4070 Basel (CH); Roche Diagnostics GmbH, 68305 Mannheim (DE)
(72) Erfinder: Frikart, Marcel, 3012 Bern (CH); Jungen, Markus, 3065 Bollingen (CH)
(74) Vertreter: Blum, Rudolf Emil

(57) **Zusammenfassung**

Zur Überwachung einer Insulinpumpe (1) ist diese zur Übertragung von Meldungen über ein Funknetz (2) kurzer Distanz an ein Smartphone (3) ausgestaltet, was mittels diesem zur automatischen Sendung einer Mitteilung (4,5,6,9) über ein Telefonnetz (6) an ein weiteres Telefongerät (8) führt. Damit kann die Insulinpumpe automatisiert fernüberwacht werden, was insbesondere bei Diabetespatienten im Kindesalter Vorteile bringt.

## Beschreibung

Die Erfindung betrifft ein Verfahren zur Überwachung eines medizinischen Gerätes, insbesondere einer Insulinpumpe und/oder eines Blutzuckermessgerätes. Ferner betrifft die Erfindung Anordnungen zur Überwachung von Insulinpumpen und/oder Blutzuckermessgeräten.

Diabetiker müssen regelmässig ihren Blutzuckerspiegel messen. Bei einer Diabetesbehandlung mit einer Insulinpumpe ist ferner die Kontrolle der abgegebenen Insulinmenge vorzunehmen. Zur Überprüfung der Werte sind vom Diabetiker bedienbare Systeme verfügbar, welche Werte von Messgerät und Infusionspumpe zusammenführen, um einen Überblick zu geben und die Werte interpretierbar zu machen. Das regelmässige Messen, die Pumpenkontrolle und die Interpretation solcher Messwerte bzw. Meldungen kann aber besonders für Kinder und ältere Patienten eine nur mit Mühe auszuführende Tätigkeit sein. Hier will die Erfindung eine Verbesserung bewirken.

Dies erfolgt gemäss dem Verfahren durch die Überwachung eines medizinischen Gerätes und insbesondere einer Insulinpumpe und/oder eines Blutzuckermessgerätes und/oder eines kontinuierlich messenden Sensors umfassend die folgenden Schritte:
- Erzeugen einer Meldung in dem medizinischen Gerät
- drahtlose Übertragung der Meldung über eine Funkstrecke kurzer Distanz an ein Kommunikationsgerät, das seinerseits zur drahtlosen Verbindungsaufnahme mit einem Telefon- oder Funkrufnetz ausgestaltet ist,
- Weiterleitung der Meldung über das Telefon-oder Funkrufnetz an ein am selben Telefon- oder Funkrufnetz angeschlossenes weiteres drahtloses Kommunikationsgerät.

Das erfindungsgemässe Verfahren erlaubt die Fernüberwachung einer Insulinpumpe und/oder eines Blutzuckermessgerätes und/oder eines kontinuierlich messenden Blutzuckersensors, z.B. durch die Eltern des Pumpenträgers oder durch professionelle Betreuer. Das drahtlose, voll automatisierte System, bei dem die Übertragung an das weitere Kommunikationsgerät durch die Insulinpumpe und/oder das Blutzuckermessgerät und/oder den Sensor angestossen wird, ermöglicht es ohne Handhabung durch den Pumpenträger und/oder Bediener des Blutzuckermessgerätes auszukommen. Für Patienten, die ohnehin ein Mobiltelefon, insbesondere ein Smartphone, oder einen Pager bei sich tragen, fällt kein Aufwand für ein zusätzliches Gerät an.

Die Aufgabe wird ferner mit den Anordnungen gemäss den Ansprüchen 12 bis 14 gelöst. Einerseits kann dabei die Insulinpumpe die Meldung drahtlos an das Mobiltelefon oder den Pager absetzen oder dies kann durch das Blutzuckermessgerät erfolgen. Bei einer Variante wird auch die Meldung des Blutzuckermessgerätes drahtlos via die Insulinpumpe an das Telefon oder den Pager abgegeben.

Im folgenden werden Ausführungsbeispiele der Erfindung unter Bezugnahme auf die Figuren näher erläutert. Dabei zeigt
Figur 1 ein Beispiel einer Meldungsübermittlung aus einer Insulinpumpe; und
Figur 2 eine besondere Ausführungsart der Meldungsübermittlung aus einem Blutzuckermessgerät.

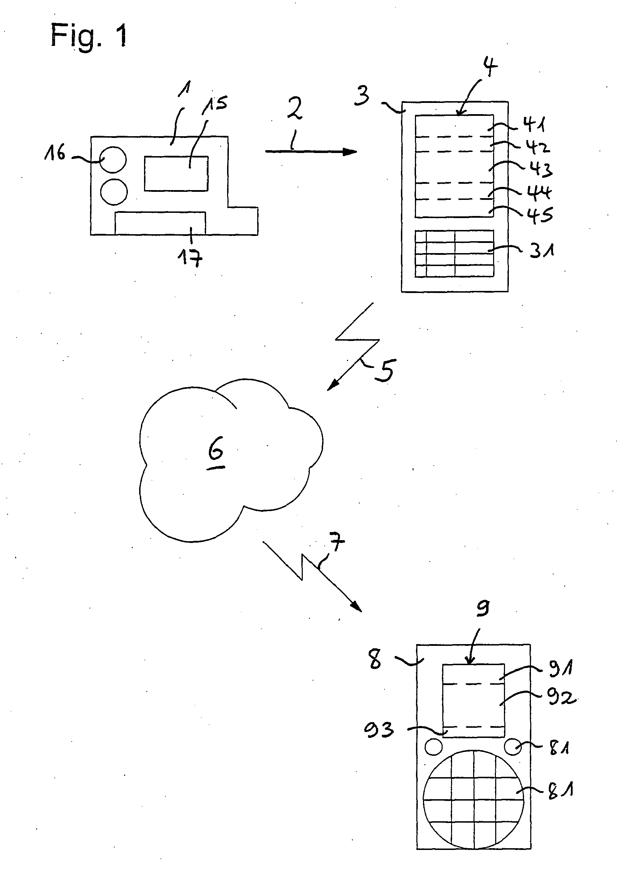

Figur 1 zeigt ein erstes Ausführungsbeispiel, bei welchem eine Insulinpumpe 1 vorgesehen ist. Die Insulinpumpe weist auf bekannte Weise eine Anzeige 15, Bedienungsmittel 16 und eine Aufnahme für die Insulinampulle auf. Derartige Insulinpumpen sind bekannt und müssen hier in Bezug auf deren Funktion zur Abgabe von Insulin an einen Patienten nicht weiter beschrieben werden. Die Insulinpumpe ist nun derart ausgestaltet, dass sie die bei bekannten Insulinpumpen vorkommenden Ereignismeldungen und/oder Alarmmeldungen über eine Funkverbindung kurzer Distanz, insbesondere eine Funkverbindung nach dem Bluetooth-Standard abgeben kann. Dies ist in der Figur mit dem Pfeil 2 dargestellt. Das erste Kommunikationsgerät 3 ist zum Empfang dieser Meldungen über die Funkverbindung kurzer Distanz ausgestaltet. Die Insulinpumpe 1 kann somit vorzugsweise über eine Bluetooth-Funkverbindung mit dem Kommunikationsgerät 3 Verbindung aufnehmen und an dieses die Infusionspumpen-Meldungen abgeben. Das Kommunikationsgerät 3 ist bevorzugterweise ein Mobiltelefon und insbesondere ein sogenanntes Smartphone, auf welchem eine entsprechende Applikation zum Empfang und zur Darstellung der Meldung von der Insulinpumpe ausgeführt wird. Derartige Smartphones sind handelsüblich und weisen bekanntermassen eine Tastatur 31 und eine Anzeige 4 auf. Ebenso ist es bekannt, dass das Mobiltelefon mit einer Bluetooth-Funktionalität bzw. einer Bluetooth-Schnittstelle versehen ist. Die Applikation nimmt die Meldung von der Insulinpumpe entgegen und generiert automatisch eine Meldung, welche über das Mobiltelefonnetz verschickt werden kann. Die Anzeige 4 zeigt dann neben dem Bereich 41, der die Symbolleiste des Betriebssystems und der Applikation zeigt z.B. den Bereich 42 auf, der den Status der Funkverbindung kurzer Distanz anzeigt und den Bereich 43, der das Senden z.B. einer SMS-Nachricht anzeigt sowie die Bereiche 44 und 45, die Meldungen und Werte der Insulinpumpe anzeigen. Mindestens eine der Meldungen der Insulinpumpe kann an das weitere Gerät 8 geschickt werden. Dies kann derart erfolgen, dass jede von der Insulinpumpe 1 an das Gerät 3 abgegebene Meldung automatisch verschickt wird oder die Applikation auf dem Gerät 3 kann so ausgestaltet sein, dass in dieser eine Entscheidung getroffen wird, ob die Meldung eine für die Fernüberwachung der Insulinpumpe 1 relevante Meldung ist, welche weitergeschickt werden soll oder ob es sich um eine andere Meldung handelt, welche lediglich auf dem Display 4 des Gerätes 3 angezeigt und allenfalls in diesem gespeichert wird. Die Applikation auf dem Gerät 3 kann dazu eine Tabelle der Meldungen einer Insulinpumpe und/oder eines Blutzuckermessgerätes enthalten, welche diese Meldungen als weiter zu leitende Meldungen und als nicht weiter zu leitende Meldungen klassiert und auf Grund einer solchen Tabelle die Entscheidung fällt, ob das Gerät 3 die Meldung an das weitere Gerät 8 weiterleitet.

Handelt es sich um eine Meldung, welche für den Fernüberwachungszweck an Dritte weitergeleitet werden soll, so wird vom Gerät 3 bzw. der auf diesem laufenden Applikationssoftware eine Meldung generiert, welche vorzugsweise als Kurzmeldung gemäss dem SMS-Protokoll oder dem MMS-Protokoll über das GSM-Netz oder UMTS-Netz 6 übermittelt wird. In der Figur ist das mit dem Übermittlungspfad 5 zum Netz 6 dargestellt. Diese Meldung kann den entsprechenden Pumpenalarm in codierter Form oder in Klartextform und/oder die Angabe des ausgeschütteten Bolus mit dem entsprechenden Wert enthalten.

Über das Mobilelefonnetz 6, oder im Falle von Pagern über das Funkrufnetz, insbesondere nach dem POCSAG Format, gelangt die Meldung an das weitere Kommunikationsgerät 8, was in der Figur mittels des Pfeils 7 dargestellt ist. Dieses Gerät 8 ist z.B. ein Mobiltelefon ohne Smartphone-Eigenschaften, ein Smartphone oder ein Pager mit einer Tastatur 81. Auf dem Display 9 mit den Bereichen 91, 92, 93 des Gerätes 8 wird dann die SMS- oder allenfalls MMS-Meldung mit dem Inhalt der Information aus der Insulinpumpe und allenfalls zusätzlichen Interpretationen und Empfehlungen für Dritte z.B. im Bereich 92 angezeigt. Die Drittperson, welche über das Gerät 8 die Meldung der Insulinpumpe erhält, kann dann entsprechend reagieren, wenn dies nötig ist. Als weitere Netze kommen die als personal network, local network oder wide area network bekannten Übermittlungsstrukturen in Frage.

Bei einer weiteren bevorzugten Ausführungsform ist der Ablauf der selbe wie bei Figur 1, doch ist das medizinische Gerät 1 in diesem Fall ein Blutzuckermessgerät oder ein kontinuierlich messender Blutzuckersensor, welches ebenfalls Alarmmeldungen und/oder Blutzuckermesswerte über das Kommunikationsgerät 3 an Dritte bzw. an das Kommunikationsgerät 8 weiter geben kann. Die Ausführung erfolgt dabei auf dieselbe Weise, wie dies anhand der Meldung der Infusionspumpe beschrieben worden ist.

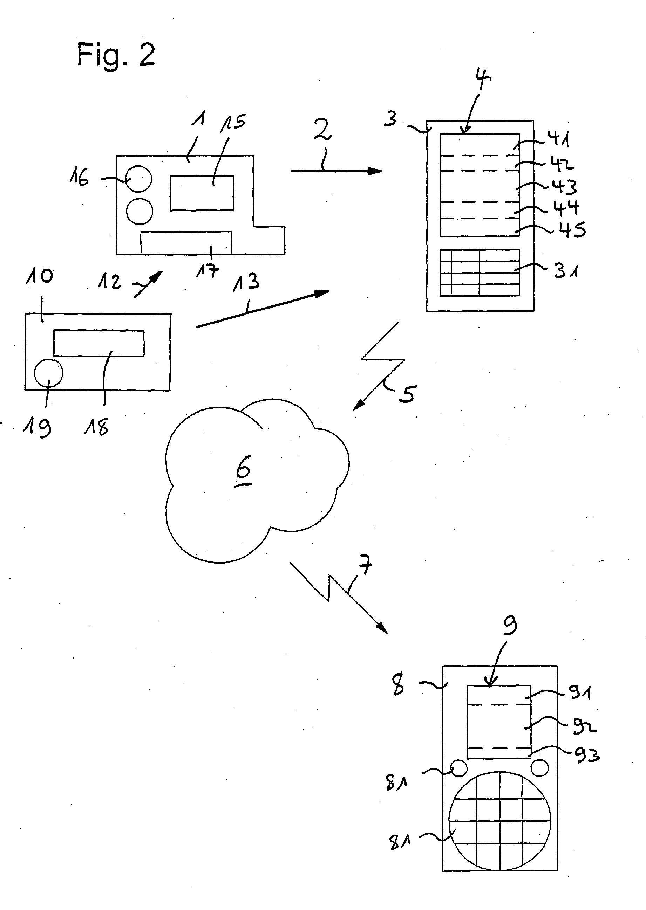

Figur 2 zeigt eine weitere Ausführungsform, bei welcher zusätzlich zu der Infusionspumpe 1 ein Blutzuckermessgerät 10, oder ein kontinuierlicher Blutzuckersensor, mit einer Anzeige 18 und Bedienelementen 19 dargestellt ist. Bei dieser Ausführungsform ist die Ausgestaltung derart, dass das Blutzuckermessgerät 10 über eine Funkverbindung kurzer Distanz, insbesondere ebenfalls eine Bluetooth-Funkverbindung, welche mit dem Pfeil 12 dargestellt ist, mit der Insulinpumpe kommuniziert. Es kann sich dabei um eine Einweg-Kommunikation von Blutzuckermessgerät zur Insulinpumpe 1 hin oder um eine Zweiweg-Kommunikation zwischen den Geräten handeln. Die Geräte tauschen dabei vorzugsweise auch Identifikationsinformationen aus, derart, dass die Insulinpumpe 1 und das Blutzuckermessgerät 10 gepaart sind und nicht auf die Informationen anderer Geräte reagieren. Ein Blutzuckermesswert oder eine Alarmmeldung des Blutzuckermessgerätes 10 wird somit an die Insulinpumpe 1 übertragen. Dort kann eine Auswertung und gegebenenfalls eine Reaktion der Insulinpumpe erfolgen, was hier aber nicht näher betrachtet wird. Relevant im Rahmen der vorliegenden Erfindung ist, dass nun die Insulinpumpe 1 entweder jede Meldung 12 des Blutzuckermessgerätes 10 über die Funkverbindung kurzer Distanz 2 an das Kommunikationsgerät 3 weiter gibt. Von diesem wird sie an das Gerät 8 weitergegeben, wobei dies für jede Meldung oder nur für ausgewählte Meldungen gelten kann, wie dies schon erläutert worden ist. Somit werden Meldungen des Blutzuckermessgerätes 10 über die Infusionspumpe 1 an die Drittperson weitergeleitet.

Bei einer anderen Ausführungsform, welche mit dem Pfeil 13 angedeutet ist, erfolgt die Kommunikation des Blutzuckermessgerätes 10 direkt mit dem Kommunikationsgerät 3.

Durch die drahtlose Anbindung, vorzugsweise über den Bluetooth-Standard, des medizinischen Gerätes 1 oder 10, welches vorzugsweise eine Insulinpumpe oder ein Blutzuckermessgerät oder ein kontinuierlich messender Sensor ist, an ein Kommunikationsgerät 3, welches vorzugsweise ein Smartphone ist, welches seinerseits an ein Funknetz 6 angeschlossen ist, kann also die Fernüberwachung auf einfache Art und Weise erfolgen. Vorteilhaft ist, dass sich ein drahtloses, voll automatisiertes System ergibt, welches keiner Bedienung durch den Träger der Infusionspumpe oder dem Benutzer des Blutzuckermessgerätes benötigt. Mit anderen Worten, die Abgabe der Meldungen und Alarme und deren Weiterleitung über das Funknetz erfolgen automatisch ohne dass eine Bedienung dafür notwendig ist. Die bevorzugte Ausgestaltung des Kommunikationsgerätes 3 als Telefon- bzw. Smartphone ergibt den Vorteil, dass keine zusätzlichen Geräte vom Patienten getragen werden müssen. Die entsprechende Fernüberwachung erlaubt einer Überwachungsperson das Feststellen, ob regelmässig gemessen wird und ob die Pumpenfunktionen ordnungsgemäss erfolgen. Dies ist besonders bei der Überwachung von Kindern nützlich, welche eine Infusionspumpe tragen.

## Patentansprüche

1. Verfahren zur Überwachung eines medizinischen Gerätes (1), umfassend die folgenden Schritte:
- Erzeugen einer Meldung in dem medizinischen Gerät
- drahtlose Übertragung der Meldung über eine Funkstrecke (2) kurzer Distanz an ein Kommunikationsgerät, das seinerseits zur drahtlosen Verbindungsaufnahme mit einem Telefon- oder Funkrufnetz (6) ausgestaltet ist,
- Weiterleitung der Meldung über das Telefon-oder Funkrufnetz an ein am selben Telefon- oder Funkrufnetz angeschlossenes weiteres drahtloses Kommunikationsgerät (8).

2. Verfahren nach Anspruch 1, **dadurch gekennzeichnet, dass** das medizinische Gerät eine Insulinpumpe (1) ist und Meldungen aufgrund eines Pumpen-Alarms und/oder einer Bolusausschüttung oder anderer Pumpenereignisse oder Ereignisse erzeugt.

3. Verfahren nach Anspruch 1, **dadurch gekennzeichnet, dass** das medizinische Gerät ein Blutzuckermessgerät ist, das Meldungen auf Grund von Blutzuckermesswerten oder anderen Messgerätereignissen oder Ereignissen erzeugt.

4. Verfahren nach Anspruch 1, **dadurch gekennzeichnet, dass** das medizinische Gerät ein kontinuierlich oder quasi-kontinuierlich arbeitender Sensor ist.

5. Verfahren nach Anspruch 2, **dadurch gekennzeichnet, dass** die Insulinpumpe (1) eine Meldung aufgrund eines zuvor an sie abgegebenen Blutzuckermesswertes erzeugt, insbesondere aufgrund eines über eine Funkstrecke (12) kurzer Distanz von einem Blutzuckermessgerät (10) an die Insulinpumpe abgegebenen Messwertes.

6. Verfahren nach einem der Ansprüche 1 bis 5, **dadurch gekennzeichnet, dass** das erste Kommunikationsgerät (3) ein drahtloses Telefon oder ein Pager ist, insbesondere ein drahtloses Telefon mit den Eigenschaften eines Computers (Smartphone).

7. Verfahren nach einem der Ansprüche 1 bis 6, **dadurch gekennzeichnet, dass** das Telefonnetz ein GSM Netz oder ein UMTS Netz oder das Funkrufnetz ein POCSAG Netz oder ein anderes Netz ist.

8. Verfahren nach einem der Ansprüche 1 bis 7, **dadurch gekennzeichnet, dass** die Meldung auf dem Telefonnetz als SMS-Meldung oder als MMS-Meldung oder über andere Übertragungsprotokolle oder Dienste übermittelt wird.

9. Verfahren nach einem der Ansprüche 1 bis 8, **dadurch gekennzeichnet, dass** das weitere Kommunikationsgerät (3) ein drahtloses Telefon oder ein Pager ist, insbesondere ein drahtloses Telefon.

10. Verfahren nach einem der Ansprüche 1 bis 9, **dadurch gekennzeichnet, dass** der Meldungsablauf automatisch erfolgt und durch das medizinische Gerät ausgelöst wird.

11. Verfahren nach Anspruch 10, **dadurch gekennzeichnet, dass** im ersten Kommunikationsgerät (3) eine Überprüfung stattfindet, ob die Meldung oder der Alarm an das weitere Kommunikationsgerät (8) weiter zu leiten ist oder nicht.

12. Anordnung zur Übertragung einer diabetesrelevanten Meldung von einem Diabetiker zu einer entfernten Person, umfassend eine Insulinpumpe (1), ausgestaltet zur Abgabe einer pumpenrelevanten Meldung über ein Funknetz kurzer Distanz (2), ein drahtloses Telefongerät (3) oder einen Pager, ausgestaltet zum Empfang einer Meldung von der Insulinpumpe über das Funknetz kurzer Distanz und zur **dadurch** automatisch ausgelösten Abgabe einer SMS-Meldung oder einer MMS-Meldung enthaltend die Information aus der Insulinpumpe an ein weiteres Telefongerät (8) oder einen Pager.

13. Anordnung zur Übertragung einer diabetesrelevanten Meldung von einem Diabetiker zu einer entfernten Person, umfassend ein Blutzuckermessgerät (10), ausgestaltet zur Abgabe einer pumpenrelevanten Meldung über ein Funknetz kurzer Distanz (2), ein drahtloses Telefongerät (3) oder einen Pager, ausgestaltet zum Empfang einer Meldung von dem Blutzuckermessgerät über das Funknetz kurzer Distanz und zur **dadurch** automatisch ausgelösten Abgabe einer SMS-Meldung oder MMS-Meldung enthaltend die Information aus dem Blutzuckermessgerät an ein weiteres Telefongerät (8) oder einen Pager.

14. Anordnung zur Übertragung einer diabetesrelevanten Meldung von einem Diabetiker zu einer entfernten Person, umfassend eine Insulinpumpe (1), ausgestaltet zum Empfang einer Meldung über ein Funknetz kurzer Distanz, ein Blutzuckermessgerät (10) ausgestaltet zur Abgabe einer Meldung über ein Funknetz (13) kurzer Distanz an die Insulinpumpe, wobei die Insulinpumpe weiter ausgestaltet ist zur Weitergabe der Meldung über ein Funknetz (2) kurzer Distanz, sowie ein drahtloses Telefongerät (3) oder einen Pager, ausgestaltet zum Empfang einer Meldung von der Insulinpumpe über das Funknetz kurzer Distanz und zur **dadurch** automatisch ausgelösten Abgabe einer SMS-Meldung oder MMS-Meldung enthaltend die Information aus der Insulinpumpe an ein weiteres Telefongerät (8) oder einen Pager.

15. Anordnung nach einem der Ansprüche 12 bis 14, **dadurch gekennzeichnet, dass** das drahtlose Telefongerät (3) oder der Pager ausgestattet ist, um eine Meldung aus der Insulinpumpe oder dem Blutzuckermessgerät daraufhin zu prüfen, ob es sich um eine weiter zu leitende Meldung handelt oder nicht, worauf die Meldung automatisch weitergeleitet oder nicht weitergeleitet wird.
